# EUROPEAN PATENT SPECIFICATION

(11) **EP 3 470 027 B1**
(45) Date of publication and mention of the grant of the patent: **30.11.2022**
(21) Application number: 18208406.1
(22) Date of filing: 30.01.2015
(51) Int. Cl.: A61F 9/007, A61F 13/38

(54) **INSTRUMENTS FOR REMOVING DEBRIS FROM AN EYE**
INSTRUMENTE ZUR ENTFERNUNG VON ABLAGERUNGEN AUS EINEM AUGE
INSTRUMENTS D'ÉLIMINATION DE DÉBRIS DANS UN IL

(43) Date of publication of application: 17.04.2019
(62) Divisional of application: 15706309.0
(73) Proprietor: Rynerson, James, M., Franklin, TN 37069 (US)
(72) Inventor: Rynerson, James, M., Franklin, TN 37069 (US)
(74) Representative: Findlay, Alice Rosemary

(56) References cited:
- EP-A1- 2 745 818
- WO-A1-2009/066077
- US-A1- 2014 052 164
- US-A1- 2014 378 878

## Description

### FIELD

The present invention relates generally to devices for removing debris from an eye to treat an ocular disorder.

### BACKGROUND

Ocular disorders such as those relating to eyelid margin disease are particularly common pathological conditions of the ocular adenexa. By way of example, these disorders include blepharitis, meibomitis, and dry eye syndrome. Despite advances in ophthalmology and medical treatments in general, until recently, the recommended treatments for these exemplary common ocular disorders has remained essentially unchanged for decades.

Historically, treatment of eyelid margin disease begins and ends with the patient. The patient first begins to notice symptoms including eyelid redness, flaking of skin on the eyelids, crusting and/or cysts at the eyelid margins, and a gritty sensation of the eye culminating in irritation, burning, and reduced vision. Should these symptoms remain unchanged or worsen, the patient routinely seeks the advice of an eye specialist, such as an ophthalmologist. After carefully considering the patients' medical history and investigating various possible causes, the specialist may prescribe a hygienic home treatment procedure for the patient to perform regularly in conjunction with antibiotics and/or topical steroids until the disease subsides.

The goal of the hygienic home treatment procedure is to remove debris, oil, and scurf that have collected along the eyelid margin during progression of the disorder. Removal of this debris is critical to both healing the eye and preventing a resurgence of the disorder. Without proper, regular removal of accumulated debris, such ocular disorders regularly worsen despite periodic treatments.

Hygienic home treatment of such ocular disorders is generally a two-step process. First, the patient softens the debris and scurf by applying a warm compress, diluted baby shampoo, or a specialized liquid solution to the eyelid margin. This first step prepares the debris for removal while preventing further irritation to the eye. Second, the patient attempts to remove the debris by physically scrubbing the eyelid margin, the base of the eyelashes, and the pores of the meibomian glands. This scrubbing is routinely attempted with either a generic cotton swab, a fingertip, or a scrub pad placed over the fingertip and applied against the eye. By cleaning debris and scurf free from the base of the eyelashes and unclogging the pores of the meibomian glands, the patient may improve the overall health of the eyelid margin; thereby reducing irritation, burning, and other symptoms related to the disorder.

Unfortunately for many patients, such hygienic home treatment is met with limited success due to the practical difficulties of cleaning one's own eye with an imprecise instrument such as a fingertip or cotton swab. For instance, many patients do not have the necessary dexterity to manipulate their fingertip or a cotton swab along the eyelid margin. Moreover, a shake, tremor, or poor near vision further complicate such self-treatment. Even for those capable of incorporating hygienic home treatment into their daily routine, many, if not most people, are wary of placing objects near their eyes to actively scrub along the eyelid margin. Given this anxiety, discomfort, and the inability to specifically target debris deposits, patients routinely fail to totally cleanse the margin of the eyelid, the base of the eyelashes, and the meibomian glands. While the attempted treatment may temporarily abate the patient's symptoms, subtle continuation of the disease often persists; thus permitting a low-grade inflammation to develop and, ultimately lead to chronic dry eye syndrome. Further, this treatment is typically required to be performed for the rest of the patient's life; thereby, creating a substantial hurdle to regular and effective compliance during hygienic home treatment.

Evidence suggests that medical costs associated with dry eye syndrome, often induced by ocular diseases such as blepharitis, are currently over 68 billion dollars each year. Many of these expenses are needlessly incurred due to the patients' failure to perform regular and effective treatments resulting in increased doctor visits, medications, and artificial tears. These expenses create a significant financial burden for insurance carriers, especially Medicare, which provides primary medical coverage for many individuals particularly prone to dry eye disease, such as the elderly.

There is a need for a method and apparatus for use at home in treating ocular disorders, such eyelid margin diseases, that addresses present challenges and characteristics such as those discussed above.

WO 2009/066077 discloses an apparatus for melting and expressing material from blocked glans in a mammalian eyelid. The apparatus includes a massager and a deformable eyelid interface with a heater. A massaging module causes the eyelid interface to massage the eyelid. The apparatus further includes a scrubbing element.

U.S. Patent Publication No. 2014/0052164 discloses an instrument for treating eyelid margin disease by removing debris from an eye during the treatment of an ocular disorder. In one exemplary embodiment, a device includes a mechanical drive, a chuck, and the instrument. The instrument includes a swab with a tip portion sized to provide access to the debris on an eyelid of an eye. The mechanical drive rotatably drives the instrument with the chuck so as to remove debris from the eye during treatment of an ocular disorder.

### SUMMARY

The invention is defined by the claims

Various additional objectives, advantages, and features of the invention will be appreciated from a review of the following detailed description of the illustrative embodiments taken in conjunction with the accompanying drawings.

### BRIEF DESCRIPTION OF THE DRAWINGS

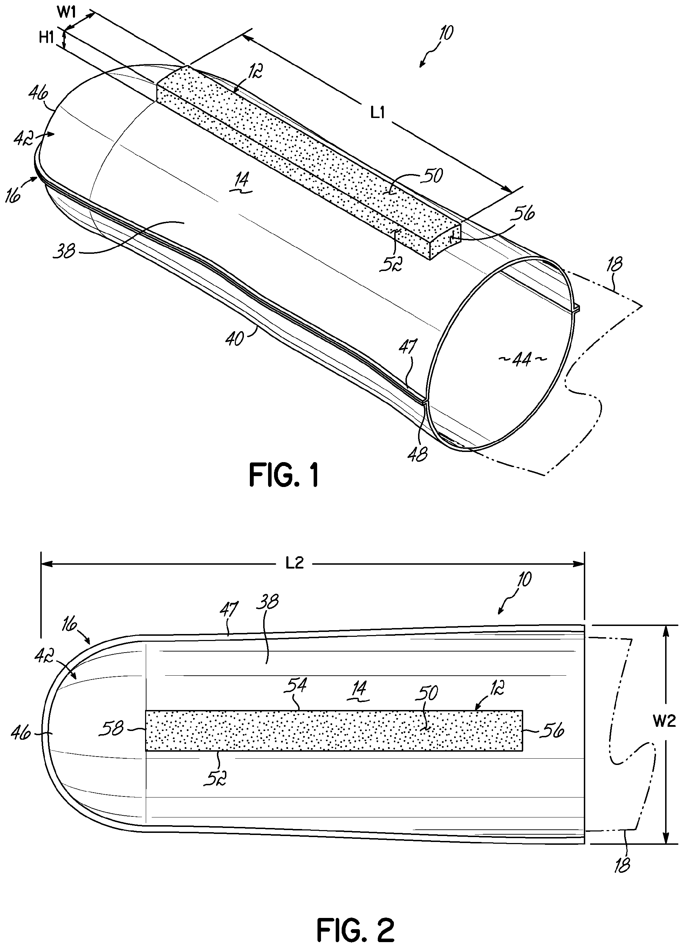

The accompanying drawings, which are incorporated in and constitute a part of this specification, illustrate embodiments of the invention and, together with a general description of the invention given above, and the detailed description given below serve to explain the invention. The examples shown in figure 1 to figure 4 are included for illustrative purpose only and do not form part of the invention.
FIG. 1 is a perspective view of an example of a device for removing debris from an eye not in accordance with the invention.
FIG. 2 is a top view of the example illustrated in FIG. 1.
FIG. 3 is a side view of the example illustrated in FIG. 1.
FIG. 4 is a cross-sectional side view of the example illustrated in FIG. 1 during use to remove debris from an eye.
FIG. 5 is a perspective view of an embodiment of a mechanical device for removing debris from an eye in accordance with aspects of the invention.
FIG. 6 is a perspective view of an embodiment of a scrubbing strip for use with a mechanical device for removing debris from an eye in accordance with aspects of the invention.
FIG. 7 is a perspective view of a rigid member for use with the scrubbing strip illustrated in FIG. 6.
FIG. 8 is a perspective view of an alternative embodiment of a rigid member having a permanently affixed scrubbing strip.
FIG. 9 is a cross-sectional side view of the embodiment illustrated in FIG. 5 during use to remove debris from an eye.

### DETAILED DESCRIPTION

With reference to FIGS. 1 to 4, an embodiment of an eye wipe device 10 for scrubbing an eyelid margin to remove debris and treating an ocular disorder includes a scrubbing strip 12 mounted to a surface 14 of a support 16 that is configured to engage a finger 18 of a user is shown (not in accord with the invention).

The scrubbing strip 12 has a length L1, a width W1, and a height H1, such that when the scrubbing strip 12 is mounted on the surface 14 of the support 16, the scrubbing strip 12 may access an inner margin 22 of the upper and lower eyelids 26, 28 of the eye 30 to contact and remove debris 32 from the inner eyelid margin 22 while minimizing, and preferably avoiding, contact with the surface 34 of the eyeball 36 when the upper and lower eyelids 26, 28 are in a closed position. While embodiments of the scrubbing strip 12 on embodiments intended for use on a finger 18 are illustrated as having a length L1 running coaxial with the user's finger, in alternative embodiments, the length of the scrubbing strip 12 may be rotated about 90 degrees so as to be aligned generally perpendicular to the length of the user's finger when in use. It will be appreciated that the scrubbing strip 12 may also access an outer margin 24 of the eyelids 26, 28 to contact and remove debris.

In the exemplary embodiment, the length L1 of the scrubbing strip 12 is aligned generally parallel to the length L2 of the support 16, which, during use, will be aligned with the length of the finger 18 of the user. The length L1 of the scrubbing strip 12 is greater than the width W2. In an embodiment, the length L1 of the scrubbing strip 12 is from about 0.75 cm to about 2 cm. In an alternative embodiment, the length L1 of the scrubbing strip 12 is from about 1 cm to about 1.75 cm. In a further alternative embodiment, the length L1 of the scrubbing strip 12 is about 1.5 cm.

In the exemplary embodiment, the width W1 of the scrubbing strip 12 is aligned generally perpendicular to the length L1 and generally parallel with the width W2 of the support 16. In an embodiment, the width W1 of the scrubbing strip 12 is from about 1 mm to about 2 mm. In an alternative embodiment, the width W1 of the scrubbing strip 12 is from about 1.25 mm to about 1.75 mm. In a further alternative embodiment, the width W1 of the scrubbing strip 12 is about 1.5 mm.

The height H1 of the scrubbing strip 12 is the distance that the scrubbing strip 12 projects from the surface 14 of the support 16. In an embodiment, the height H1 of the scrubbing strip 12 is from about 0.75 mm to about 1.5 mm. In an alternative embodiment, the height H1 of the scrubbing strip 12 is from about 0.9 mm to about 1.2 mm. In a further alternative embodiment, the height H1 of the scrubbing strip 12 is about 1 mm.

In the exemplary embodiment, the scrubbing strip 12 has an upper surface 50 and a lower surface. The lower surface is not shown as it is blocked from view by the support 16. The exemplary scrubbing strip 12 also has oppositely disposed side surfaces 52, 54 that are generally parallel to the length L1 of the scrubbing strip 12. The scrubbing strip 12 may also have oppositely disposed end surfaces 56, 58 that are generally parallel to the width W1 of the scrubbing strip 12. The upper surface 50 of the scrubbing strip 12 is illustrated as being generally planar, however, the upper surface 50 may also take other shapes, such as forming a convex curve between the side surfaces 52, 54, the end surfaces 56, 58, or both the side and end surfaces.

It will be appreciated that the scrubbing strip 12 may be manufactured of any material suitable for contacting parts of the eye 30 without harming the eye 30 including the inner and outer margins 22, 24 of the upper and lower eyelids 26, 28, as well as the surface 34 of the eyeball 36. However, as shown in the embodiment of FIGS. 1-4, the scrubbing strip 12 is a sponge. As described herein, "sponge" broadly refers to any material that is soft, porous, and resilient. Particularly, the scrubbing strip 12 is a medical grade sponge or a surgical grade sponge capable of removing debris from parts of eye 30 without harming the eye 30. As shown in the exemplary embodiment of FIGS. 1-4, the scrubbing strip 12 is selected from a polyvinyl alcohol (PVA) sponge or a methyl cellulose sponge. It will be appreciated, however, that similar materials capable of removing debris from on the eye 30 without harming the eye 30 are readily apparent and may also be used.

In the embodiment illustrated in FIGS. 1-4, the support 16 is configured to placed over the end of a finger 18, illustrated with broken lines, with the scrubbing strip 12 positioned over the pad of the finger tip. The exemplary embodiment illustrated in FIGS. 1-4 includes a first sheet 38 coupled to a second sheet 40 to form a sleeve 42 that includes an opening 44 in one end for insertion of the finger 18. A closed end 46 is distal to the opening 44, however, it is anticipated that in some embodiments, the both ends may be open. The first and second sheets 38, 40 are coupled to one another around the majority of their respective edges 47, 48. The first and second sheets 38, 40 may be coupled to one another using known techniques suitable to the materials from which the sheets are made. For example, the first and second sheets 38, 40 may be attached to one another with stitching, an adhesive, welding, and combinations thereof. It will be appreciated that the support 16 may be formed without using multiple sheets. For example, the support could be formed from a tubular material or from a single length of material that is folded over on itself such that opposite edges of the sheet are brought together and coupled to one another.

The support 16 may be composed of any material having sufficient structural integrity to support the scrubbing strip 12 and that is safe for use around the eye 30. Exemplary materials include a woven material, a knit material, non-woven/non-knit fiber based material, a polymeric material, and combinations thereof. Elastic materials may also be used in aspects of the support 16, such as for the second sheet 40. In an embodiment, the first and second sheets are formed from a non-woven/non-knit polyester material.

The lower surface of the scrubbing strip 12 may be attached to the outer surface 14 of the support 16 with an adhesive that is safe for use on devices that come in contact with the eye 30. It is anticipated that the scrubbing strip 12 may be attached to the outer surface 14 of the support 16 with other techniques, such as stitching.

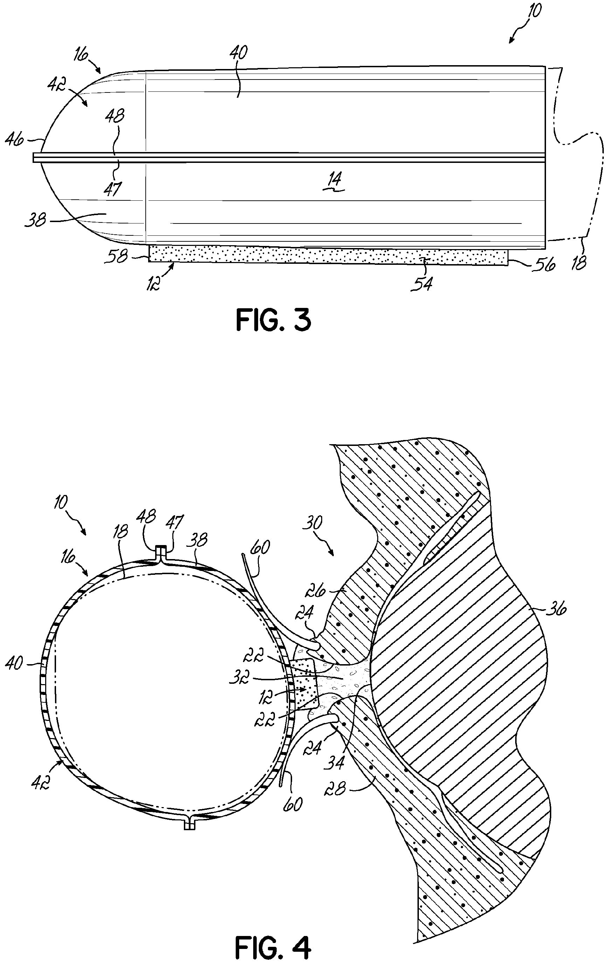

As illustrated in FIG. 4, during use, the support 16 is fit over the end of a finger 18 such that the scrubbing strip 12 is positioned over the pad of the finger tip. The scrubbing strip 12 may optionally be impregnated or soaked in a liquid, such as a cleaning solution suitable for use with the eye, prior to the user bringing the finger 18 with eye wipe device 10 into close proximity with the eye 30 to be scrubbed. The eye wipe device 10 may be used by the subject to scrub the subject's own eyelid margin or by a caregiver to scrub the subject's eyelid margin. The subject closes the eye 30 to be scrubbed and the scrubbing strip 12 is positioned adjacent the eye 30 generally parallel to the closed eyelids 26, 28 of the eye 30 to be scrubbed. The scrubbing strip 12 is then brought into contact with the eye 30, and preferably with the inner eyelid margin 22 between the eyelashes 60 of the upper eyelid 26 and lower eyelid 28 such that the scrubbing strip 12 contacts and removes debris 32 along the inner margins 22 of the eyelids 26, 28. The height H1 of the scrubbing strip 12 works in combination with the surface 14 to prevent or reduce the likelihood of the scrubbing strip 12 contacting the surface 34 of the eyeball 36. The scrubbing strip 12 may also be brought into contact with the outer margins 24 of the eyelids 26, 28 to remove debris.

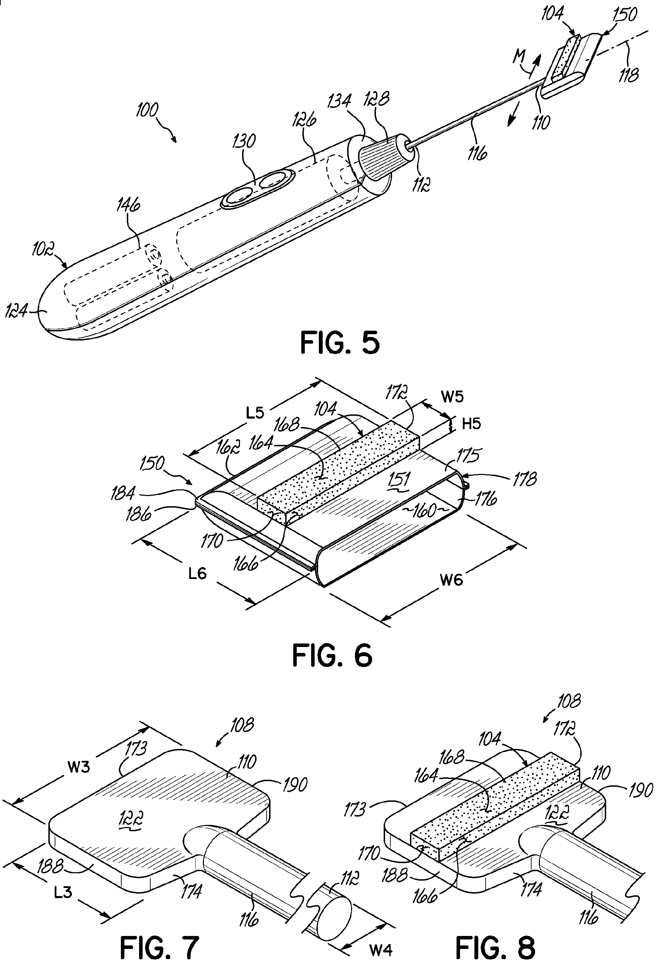

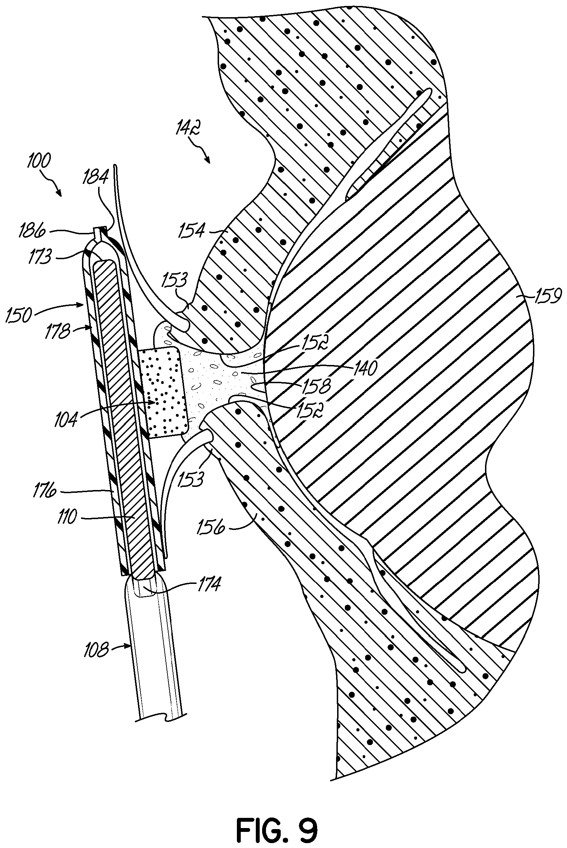

With reference to FIGS. 5-9, another aspect of the invention is directed to a mechanical eye wipe device 100 for scrubbing an eyelid margin to remove debris and treating an ocular disorder. The mechanical eye wipe device 100 includes a mechanical drive unit 102 which operably moves a scrubbing strip 104 to facilitate removal of debris 140 from an eye 142 (See FIG. 9). The scrubbing strip 104 is coupled to a rigid member 108 having both a distal end portion 110 and a proximal end portion 112. The scrubbing strip 104 is coupled to the distal end portion 110 of the rigid member 108. In an embodiment, the proximal end portion 112 of the rigid member 108 is secured to the mechanical drive unit 102 in order to transmit motion from the mechanical drive unit 102, through the rigid member 108, and to the scrubbing strip 104. In an embodiment, the proximal end portion 112 of the rigid member 108 is permanently secured to the mechanical drive unit 102. In another embodiment, the proximal end portion 112 of the rigid member 108 is removably secured to the mechanical drive unit 102. It will be appreciated that any known method may be used to secure, permanently or removably, the rigid member 108 to the mechanical drive unit 102.

The rigid member 108 includes a shaft 116 and a central axis 118. The shaft 116 extends along the central axis 118 between the proximal end portion 112, which is coupled to the mechanical drive unit 102 and the distal end portion 110 having a scrubbing strip 104 coupled thereto. The rigid member 108 is sufficiently rigid to effectively transmit motion from the mechanical drive unit 102, to the distal end portion 110 and thereby, the scrubbing strip 104. In an embodiment, the rigid member 108 is made from a plastic material, however, other sufficient rigid materials may also be used.

In the exemplary embodiments shown in FIGS. 5, 7, and 8, the distal end portion 110 of the rigid member 108 has a generally planar surface 122 having a width W3 that is greater than a width W4 of the shaft 116 of the rigid member 108. While the surface 122 of the distal end portion 110 is illustrated as being generally planar, in alternative embodiments of the invention, the surface 122 may be a generally curved surface. For example, the surface 122 may be a convex curved surface with the distal and proximal edges 173, 174 or the left and right edges 188, 190 angled away from the eye so as to prevent the respective edges from contacting structures of the eye during treatment. The distal end portion 110 also has a length L3 that is coaxial with the central axis 118 of the shaft 116 of the rigid member 108. The distal end portion 110 may be generally paddle-shaped. As exemplified in FIGS. 7 and 8, the paddle-shaped distal end portion 110 may be generally rectangular. However, other shapes may be used for the distal end portion 110 so long as the other shapes provide sufficient surface area for the scrubbing strip 104.

In the exemplary embodiment, the width W3 of the distal end portion 110 of the rigid member 108 is at least as long as the length L5 of the scrubbing strip 104. Preferably, the width W3 of the distal end portion 110 is equal or slightly greater than (i.e., not more than about 20% greater, and preferably, not more than 10% greater than) the length L5 of the scrubbing strip. Accordingly, in an embodiment, the width W3 of the distal end portion 110 of the rigid member 108 is from about 0.75 cm to about 2.4 cm. In an alternative embodiment, the width W3 of the distal end portion 110 of the rigid member 108 is from about 0.75 cm to about 2.2 cm. In another alternative embodiment, the width W3 of the distal end portion 110 of the rigid member 108 is from about 1 cm to about 2 cm. In another alternative embodiment, the width W3 of the distal end portion 110 of the rigid member 108 is from about 1.5 cm to about 1.8 cm.

In the exemplary embodiment, the length L3 of the distal end portion 110, as measured between the distal edge 173 and the proximal edge 174 of the distal end portion 110, is greater than the width W5 of the scrubbing strip 104. In an embodiment, the length L3 of the distal end portion 110 is at least two times greater than the width W5 of the scrubbing strip. In an alternative embodiment, the length L3 of the distal end portion 110 is at least three times greater than the width W5 of the scrubbing strip. In an embodiment, the length L3 of the distal end portion 110 is from about 2 mm to about 20 mm. In another embodiment, the length L3 of the distal end portion 110 is from about 5 mm to about 15 mm. In another embodiment, the length L3 of the distal end portion 110 is from about 6 mm to about 10 mm.

The mechanical drive unit 102 includes a body 124, an electric motor 126, a chuck 128, and a control switch 130. As such, the device 100 is electromechanical in nature. In an exemplary embodiment, the electric motor 126, the chuck 128, and the control switch 130 are integrated into the body 124 so that the mechanical device 100 is configured to be handheld as shown in FIG. 5. However, the mechanical device 100 is not intended to be limited to a handheld configuration, and it will be appreciated that other configurations of the device 100 are readily apparent.

According to the present embodiment, the electric motor 126 is positioned within the body 124. The chuck 128 is operably connected to the electric motor 126 at a forward end portion 134 of the body 124. The proximal end portion 112 of the rigid member 108 is removably secured to the chuck 128. As described herein, the chuck 128 is generally any element capable of securing the rigid member 108 to the mechanical drive unit 102. In embodiments utilizing a removable rigid member, the chuck 128 may be tightened or loosened to respectively secure or remove the removable rigid member to the chuck 128. Through the chuck 128, the operable connection of the electric motor 126 transmits a movement to the rigid member 108. The movement is any motion relative to the mechanical drive unit 102 or, more particularly, to the body 124, that creates relative motion to the debris 140 on the eye such that upon contacting the debris 140 with the scrubbing strip 104, the debris 140 is removed. The mechanical drive unit 102 creates relative motion M in the distal end portion 110 of the rigid member 108 that is in the same general direction as the length L5 of the scrubbing strip 104 coupled to the distal end portion. In embodiments in which the scrubbing strip 104 is coupled to the distal end portion 110 with the length L5 of the scrubbing strip 104 extending in a direction generally perpendicular to the central axis 118 of the shaft 116, the mechanical drive unit 102 creates a side to side movement M that is generally perpendicular to the central axis 118 of the shaft 116. This alternative embodiment is not illustrated herein. In alternative embodiments in which the scrubbing strip 104 may be coupled to the distal end portion 110 with the length L4 extending in a direction generally parallel to the central axis 118 of the shaft 116, the mechanical drive unit creates an in and out movement that is generally parallel to the central axis 118 of the shaft 116. Thus, the movement may be a vibrating movement either orthogonal to the central axis 118 of the rigid member 108 or along the central axis 118 of the rigid member 108. In addition, the speed of the movement of the scrubbing strip 104 is any speed sufficient to remove debris 140 from on the eye 142. It will be appreciated that the speed discussed herein collectively refers to both relative speed of the scrubbing strip 104 and the frequency of the movement of the scrubbing strip 104. For instance, the frequency may range from sonic frequencies to ultrasonic frequencies. Furthermore, the speed of the scrubbing strip 104 may be variable or otherwise selectable such that an operator of the device 100 may select a desirable speed via the control switch 130.

Moreover, the control switch 130 is operably connected to the electric motor 126 and an electric power source 146 to power the device 100 on and off. In an exemplary embodiment, the electric power source 146 is a battery power source contained within the body 124. The battery power source may be either disposable or rechargeable. The electric power source 146 operably provides electrical power to the electric motor 126, which the operator controls via the control switch 130. It will be appreciated that any known control switch or plurality of control switches may be configured to power the device 100 on and off.

Furthermore, it will be appreciated that the device 100 may be manufactured from various materials suited to specific environments of use. For instance, operators may desire a durable, reusable mechanical drive unit 102 with permanently attached rigid members 108 but with single use scrubbing strips 104 shown in FIGS. 5 and 6 mounted on a removable support 150. In the alternative, operators may desire a durable, reusable mechanical drive unit 102 with removably attached rigid members 108. In this latter embodiment, the scrubbing strip 104 may be permanently mounted on the distal end portion 110 and the rigid member 108 may be suitable for a single use (as shown in FIG. 8), or the scrubbing strip 104 may be mounted on a removable support 150 and such that the rigid member 108 may be utilized more than once (as shown in FIGS. 5 and 6).

The scrubbing strip 104 for use on the mechanical eye wipe device 100 is similar to the scrubbing strip 12 utilized with the eye wipe device 10 described herein with respect to the embodiment illustrated in FIGS. 1-4.

In particular the scrubbing strip 104 has a length L5, a width W5, and a height H5, such that when the scrubbing strip 104 is mounted on the surface 151 of support 150 (FIG. 6) or on the surface 122 of the distal end portion 110 of the rigid member 108 (FIG. 8), the scrubbing strip 104 may access an inner margin 152 of the upper and lower eyelids 154, 156 of the eye 142 to contact and remove debris 140 from the inner eyelid margin 152 while minimizing, and preferably avoiding, contact with the surface 158 of the eyeball 159 when the upper and lower eyelids 154, 156 are in a closed position. The length L5 of the scrubbing strip 104 is aligned generally parallel to the width W6 of the support 150, which, during use, will be generally perpendicular to the central axis 118 of the shaft 116 of the rigid member 108. While embodiments of the scrubbing strip 104 are illustrated as having a length L5 running generally perpendicular to the central axis 118 of the shaft 116 of the rigid member 108, in alternative embodiments, the length L5 of the scrubbing strip 104 may be rotated about 90 degrees so as to be aligned generally parallel to the central axis 118.

The length L5 is greater than the width W4. In an embodiment, the length L5 of the scrubbing strip 104 is from about 0.75 cm to about 2 cm. In an alternative embodiment, the length L5 of the scrubbing strip 104 is from about 1 cm to about 1.75 cm. In a further alternative embodiment, the length L5 of the scrubbing strip 104 is about 1.5 cm.

The width W5 of the scrubbing strip 104 is aligned generally perpendicular to the length L5 and generally parallel to the length L6 the surface 151 of the support 150. In an embodiment, the width W5 of the scrubbing strip 104 is from about 1 mm to about 2 mm. In an alternative embodiment, the width W5 of the scrubbing strip 104 is from about 1.25 mm to about 1.75 mm. In a further alternative embodiment, the width W5 of the scrubbing strip 104 is about 1.5 mm.

The height H5 of the scrubbing strip 104 is the distance that the scrubbing strip 104 projects from the surface 151 of the support 150 (FIG. 6) or from the surface 122 of the distal end portion 110 (FIG. 8). In an embodiment, the height H5 of the scrubbing strip 104 is from about 0.75 mm to about 1.5 mm. In an alternative embodiment, the height H5 of the scrubbing strip 104 is from about 0.9 mm to about 1.2 mm. In a further alternative embodiment, the height H5 of the scrubbing strip 104 is about 1 mm.

The scrubbing strip 104 has an upper surface 164 and a lower surface, which is not shown as it is block from view by the support 150 (FIG. 6) or the surface 122 of the distal end portion 110 (FIG. 8). The scrubbing strip 104 may also have oppositely disposed side surfaces 166, 168 defining the length L4 of the scrubbing strip 104. The scrubbing strip 104 may also have oppositely disposed end surfaces 170, 172 defining the width W5 of the scrubbing strip 104. The upper surface 164 of the scrubbing strip 104 is illustrated as being generally planar, however, the upper surface 164 may also take other shapes, such as forming a convex curve between the side surfaces 166, 168, the end surfaces 170, 172, or both the side and end surfaces.

It will be appreciated that the scrubbing strip 104 may be manufactured of any material suitable for contacting parts of the eye 142 without harming the eye 142 including the inner and outer margins 152, 154 of the upper and lower eyelids 154, 156 as well as the surface 158 of the eyeball 159. As shown in the embodiment of FIGS. 5-9, the scrubbing strip 104 is a sponge.

In the exemplary embodiment illustrated in FIGS. 5, 6, and 9, the support 150 is configured to placed over the distal end portion 110 of the rigid member 108 with the scrubbing strip 104 positioned generally equidistant between the distal edge 173 and proximal edge 174 of the surface 122 of the distal end portion 108. The exemplary embodiment illustrated in FIGS. 5, 6, and 9 includes a first sheet 175 coupled to a second sheet 176 to form a sleeve 178 that includes an opening 160 in one end for insertion of the distal end portion 110 of the rigid member 108. A closed end 162 is distal to the opening 160. The first and second sheets 175, 176 are coupled to one another around the majority of their respective edges 184, 186. The first and second sheets 175, 176 may be coupled to one another using known techniques suitable to the materials from which the sheets are made. It will be appreciated that the support 16 may be formed without using multiple sheets. For example, the support could be formed from a tubular material or from a single length of material that is folded over on itself such that opposite edges of the sheet are brought together and coupled to one another.

As illustrated in the exemplary embodiment shown in FIG. 5, the support 150 has a size sufficient to fit over the distal end portion 110 of the rigid member 108. In an embodiment, the support 150 is sized so that its internal surfaces engage the external surfaces of the distal end portion 110 to maintain the support 150 in position on the distal end portion 110 during use. The internal surfaces of the support 150, the external surfaces of the distal end portion 110, or both the internal surfaces of the support 15 and the external surfaces of the distal end portion 110 may further include a treatment that either maintains or assists with maintaining the position of the support 150 on the distal end portion 110. For example, the respective surfaces may be treated with an adhesive or a friction increasing treatment such as a tacky coating or a treatment that roughens the surfaces. While reference is made to the internal surfaces of the support or the external surfaces of the distal end portion 110, it is anticipated that just one surface may be treated, or a portion of one or more surfaces may be treated.

The support 150 may be composed of any material having sufficient structural integrity to support the scrubbing strip 104 and that is safe for use around the eye 142. Exemplary materials include a woven material, a knit material, non-woven/non-knit fiber based material, a polymeric material and combinations thereof. Elastic materials may also be used in aspects of the support 150, such as for the second sheet 176. In an embodiment, the first and second sheets are formed from a non-woven/non-knit polyester material.

The lower surface of the scrubbing strip 104 may be attached to the outer surface 151 of the support 150 with an adhesive that is safe for use on devices that come in contact with the eye. The scrubbing strip 104 may be attached to the outer surface 151 of the support 150 with other techniques, such as stitching.

As illustrated in FIGS. 9, during use, the support 150 is fit over the distal end portion 110 of the rigid member such that the scrubbing strip 104 is generally equidistant between the distal and proximal edges 173, 174 of the surface 122 of the distal end portion 110. The scrubbing strip 12 may optionally be impregnated or soaked in a liquid, such as a cleaning solution, prior to the user bringing the distal end portion 110 into close proximity with the eye 142 to be scrubbed. The mechanical eye wipe device 100 may be used by the subject to scrub the subject's own eyelid margin or by a caregiver to scrub the subject's eyelid margin. The subject closes the eye 142 to be scrubbed and the scrubbing strip 104 is positioned adjacent the eye 142 generally parallel to the closed eyelids 154, 156 of the eye 142 to be scrubbed. The scrubbing strip 104 is then brought into contact with the eye 142, and preferably with the eyelid margin 152 between the eyelashes of the upper eyelid 154 and lower eyelid 156 such that the scrubbing strip 104 contacts and removes debris 140 along the inner margin 152 of the eyelids 154, 156. The height H5 of the scrubbing strip 104 works in combination with the surface 151 to prevent or reduce the likelihood of the scrubbing strip 104 contacting the surface 158 of the eyeball 159. The scrubbing strip 104 may also be brought into contact with the outer margin 153 of the eyelids 154, 156 to remove debris.

While the present invention has been illustrated by the description of one or more embodiments thereof, and while the embodiments have been described in considerable detail, additional advantages and modifications will readily appear to those skilled in the art. For example, while embodiments of the scrubbing strip on embodiments intended for use on a finger are illustrated as having a length running coaxial with the length of the user's finger, in alternative embodiments, the length of the scrubbing strip may be rotated about 90 degrees so as to be aligned generally perpendicular to the length of the user's finger when in use. Likewise, while embodiments of the scrubbing strip intended for use with a mechanical device are illustrate as running perpendicular to the central axis of the rigid member shaft, in alternative embodiments, the length of the scrubbing strip may be rotated about 90 degrees so as to be aligned generally parallel to the central axis. In this alternative embodiment, the device will move the scrubbing strip generally parallel to the central axis of the rigid member. Furthermore, embodiments of the scrubbing strip 12 on embodiments intended for use on a finger 18 are illustrated as having a length L1 extending only partially along the length L2 of the support 16, i.e., the scrubbing strip 12 does not extend along its length L2 all the way to either the closed end 46 or the end with the opening 44 of the support
16. However, it is anticipated that the scrubbing strip 12 could extend all the way to, or nearly all the way to the closed end 46, the end with the opening 44, or both the closed end 46 and end with the opening 44 of the support 12.

## Claims

1. A mechanical device (100) for the removal of a debris from an eye, comprising;
a rigid member (108) having a shaft (116) with a central axis (118), said rigid member (108) having a rectangular distal end portion (110) and a proximal end portion (112), said distal end portion (110) having a surface (122) with a width (W3) extending perpendicular to the central axis (118) and a length (L3) extending parallel to the central axis (118), the width (W3) being greater than the length (L3), the width (W3) being greater than a width (W4) of the shaft (116) of the rigid member (108);
a scrubbing strip (104) coupled to the distal end portion (110) and having a length (L5), a width (W5), and a height (H5), the length (L5) being greater than the width (W5); and
a mechanical drive unit (102) having a body (124), said mechanical drive unit (102) being secured to said proximal end portion (112) of said rigid member (108),
wherein said mechanical drive unit (102) operably moves said scrubbing strip (104) in a direction perpendicular or parallel to the central axis (118) of the shaft (116) facilitating removal of the debris from on the eye (30).

2. The device (100) of claim 1, wherein the length (L5) of the scrubbing strip (104) is from 0.75 cm to 2 cm, the width (W5) of the scrubbing strip (104) is from 1 mm to 2mm, and the height (H5)of the scrubbing strip (104) projects from the surface (122) from 0.75 mm to 1.5 mm.

3. The device (100) of either claim 1 or claim 2, wherein the scrubbing strip (104) is attached directly to the surface (122) of the distal end portion (110).

4. The device (100) of either claim 1 or claim 2, wherein the scrubbing strip (104) is mounted on a support (150) and the support (150) is coupled to the distal end portion (110).

5. The device (100) of claim 4, wherein the support (150) has a length (L6) extending parallel to the central axis (118) and a width (W6) extending perpendicular to the central axis (118), the length (L6) being greater than or equal to the width (W6) and the scrubbing strip (104) is positioned on the support (150) such that the length (L5) of the scrubbing strip (104) is parallel to the length (L5) of the support (150) or perpendicular to the length (L5) of the support (150).

6. The device (100) of either claim 4 or claim 5, wherein the support (150) is in the form of a sleeve (178) having an open end (160).

7. The device (100) of claim 6, wherein the open end (160) is parallel to the direction of the length (L5) of the scrubbing strip (104) or the open end (160) is perpendicular to the direction of the length (L5) of the scrubbing strip (104).

8. The device (100) of any preceding claim, wherein said mechanical drive unit (102) further includes:
an electric motor (126);
a chuck (128), said chuck (128) operably connected to said electric motor (126); and
a control switch (130), said control switch (130) operably coupled to the electric motor (126) to turn the electric motor (126) on or off,
wherein said proximal end portion (112) of said rigid member (108) is secured to said chuck (128).

9. The device (100) of claim 8, wherein said proximal end portion (112) of said rigid member (108) is removably secured to said chuck (128).

10. The device (100) of any preceding claim, further including an electric power source (146), said electric power source (146) operably coupled to said mechanical drive unit (102) to drive the movement of said distal end portion (110).

11. The device (100) of claim 10, wherein said electric power source (146) is a battery.

12. The device (100) of any preceding claim, wherein said mechanical drive unit (102) is configured to be handheld.

13. The device (100) of any preceding claim, wherein the surface (122) of the distal end portion (110) of the rigid member (108) is planar or is a curved surface.

## Patentansprüche

1. Mechanische Vorrichtung (100) zur Entfernung von Ablagerungen von einem Auge, die Folgendes beinhaltet:
ein starres Element (108), das eine Welle (116) mit einer Mittelachse (118) aufweist, wobei das genannte starre Element (108) einen rechteckigen distalen Endteil (110) und einen proximalen Endteil (112) aufweist, wobei der genannte distale Endteil (110) eine Oberfläche (122) mit einer Breite (W3), die sich lotrecht zur Mittelachse (118) erstreckt, und einer Länge (L3), die sich parallel zur Mittelachse (118) erstreckt, aufweist, wobei die Breite (W3) größer als die Länge (L3) ist, die Breite (W3) größer als eine Breite (W4) der Welle (116) des starren Elements (108) ist;
einen Abreibstreifen (12), der mit dem distalen Endteil (110) gekoppelt ist und eine Länge (L5), eine Breite (W5) und eine Höhe (H5) aufweist, wobei die Länge (L5) größer ist als die Breite (W5); und
eine mechanische Antriebseinheit (102), die einen Körper (124) aufweist, wobei die mechanische Antriebseinheit (102) an dem genannten proximalen Endteil (112) des genannten starren Elements (108) befestigt ist,
wobei die genannte mechanische Antriebseinheit (102) den genannten Abreibstreifen (104) funktionell in einer zur Mittelachse (118) der Welle (116) lotrechten oder parallelen Richtung bewegt, so dass die Entfernung von Ablagerungen von dem Auge (30) ermöglicht wird.

2. Vorrichtung (100) nach Anspruch 1, wobei die Länge (L5) des Abreibstreifens (104) von 0,75 cm bis 2 cm ist, die Breite (W5) des Abreibstreifens (104) von 1 mm bis 2 mm ist und die Höhe (H5) des Abreibstreifens (104) von 0,75 mm bis 1,5 mm von der Oberfläche (122) vorsteht.

3. Vorrichtung (100) nach Anspruch 1 oder Anspruch 2, wobei der Abreibstreifen (104) direkt an der Oberfläche (122) des distalen Endteils (110) angebracht ist.

4. Vorrichtung (100) nach Anspruch 1 oder Anspruch 2, wobei der Abreibstreifen (104) an einem Träger (150) montiert ist und der Träger (150) mit dem distalen Endteil (110) gekoppelt ist.

5. Vorrichtung (100) nach Anspruch 4, wobei der Träger (150) eine Länge (L6), die sich parallel zur Mittelachse (118) erstreckt, und eine Breite (W6), die sich lotrecht zur Mittelachse erstreckt, aufweist, wobei die Länge (L6) größer oder gleich der Breite (W6) ist und der Abreibstreifen (104) so auf dem Träger (150) positioniert ist, dass die Länge (L5) des Abreibstreifens (104) parallel zur Länge (L5) des Trägers (150) oder lotrecht zur Länge (L5) des Trägers (150) ist.

6. Vorrichtung (100) nach Anspruch 4 oder Anspruch 5, wobei der Träger (150) die Form einer Hülse (178) mit einem offenen Ende (160) hat.

7. Vorrichtung (100) nach Anspruch 6, wobei das offene Ende (160) parallel zur Richtung der Länge (L5) des Abreibstreifens (104) ist oder das offene Ende (160) lotrecht zur Richtung der Länge (L5) des Abreibstreifens (104) ist.

8. Vorrichtung (100) nach einem der vorhergehenden Ansprüche, wobei die genannte mechanische Antriebseinheit (102) ferner Folgendes beinhaltet:
einen Elektromotor (126);
ein Spannfutter (128), wobei das genannte Spannfutter (128) funktionell mit dem genannten Elektromotor (126) verbunden ist; und
einen Bedienschalter (130), wobei der genannte Bedienschalter (130) zum Ein- oder Ausschalten des Elektromotors (126) funktionell mit dem Elektromotor (126) gekoppelt ist,
wobei der genannte proximale Endteil (112) des genannten starren Elements (108) an dem genannten Spannfutter (128) befestigt ist.

9. Vorrichtung (100) nach Anspruch 8, wobei der genannte proximale Endteil (112) des genannten starren Elements (108) abnehmbar an dem genannten Spannfutter (128) befestigt ist.

10. Vorrichtung (100) nach einem der vorhergehenden Ansprüche, die ferner eine elektrische Energiequelle (146) hat, wobei die genannte elektrische Energiequelle (146) zum Antreiben der Bewegung des genannten distalen Endteils (110) funktionell mit der genannten mechanischen Antriebseinheit (102) gekoppelt ist.

11. Vorrichtung (100) nach Anspruch 10, wobei die genannte elektrische Energiequelle (146) eine Batterie ist.

12. Vorrichtung (100) nach einem der vorhergehenden Ansprüche, wobei die genannte mechanische Antriebseinheit (102) gestaltet ist, um in der Hand gehalten zu werden.

13. Vorrichtung (100) nach einem der vorhergehenden Ansprüche, wobei die Oberfläche (122) des distalen Endteils (110) des starren Elements (108) eben oder eine gekrümmte Oberfläche ist.

## Revendications

1. Dispositif mécanique (100) pour enlever un débris d'un œil, comprenant :
un membre rigide (108) ayant un arbre (116) avec un axe central (118), ledit membre rigide (108) ayant une partie d'extrémité distale rectangulaire (110) et une partie d'extrémité proximale (112), ladite partie d'extrémité distale (110) ayant une surface (122) avec une largeur (W3) s'étendant perpendiculaire à l'axe central (118) et une longueur (L3) s'étendant parallèle à l'axe central (118), la largeur (W3) étant plus grande que la longueur (L3), la largeur (W3) étant plus grande qu'une largeur (W4) de l'arbre (116) du membre rigide (108) ;
une bande de nettoyage (104) couplée à la partie d'extrémité distale (110) et ayant une longueur (L5), une largeur (W5) et une hauteur (H5), la longueur (L5) étant plus grande que la largeur (W5) ; et
une unité d'entraînement mécanique (102) ayant un corps (124), ladite unité d'entraînement mécanique (102) étant fixée à ladite partie d'extrémité proximale (112) dudit membre rigide (108),
dans lequel ladite unité d'entraînement mécanique (102) déplace de manière opérationnelle ladite bande de nettoyage (104) dans une direction perpendiculaire ou parallèle à l'axe central (118) de l'arbre (116) facilitant l'enlèvement du débris sur l'œil (30).

2. Dispositif (100) selon la revendication 1, dans lequel la longueur (L5) de la bande de nettoyage (104) est de 0,75 cm à 2 cm, la largeur (W5) de la bande de nettoyage (104) est de 1 mm à 2 mm, et la hauteur (H5) de la bande de nettoyage (104) fait saillie de la surface (122) de 0,75 mm à 1,5 mm.

3. Dispositif (100) selon la revendication 1 ou la revendication 2, dans lequel la bande de nettoyage (104) est attachée directement à la surface (122) de la partie d'extrémité distale (110).

4. Dispositif (100) selon la revendication 1 ou la revendication 2, dans lequel la bande de nettoyage (104) est montée sur un support (150) et le support (150) est couplé à la partie d'extrémité distale (110).

5. Dispositif (100) selon la revendication 4, dans lequel le support (150) a une longueur (L6) s'étendant parallèle à l'axe central (118) et une largeur (W6) s'étendant perpendiculaire à l'axe central (118), la longueur (L6) étant plus grande ou égale à la largeur (W6) et la bande de nettoyage (104) est positionnée sur le support (150) de telle sorte que la longueur (L5) de la bande de nettoyage (104) est parallèle à la longueur (L5) du support (150) ou perpendiculaire à la longueur (L5) du support (150).

6. Dispositif (100) selon la revendication 4 ou la revendication 5, dans lequel le support (150) est en la forme d'une gaine (178) ayant une extrémité ouverte (160).

7. Dispositif (100) selon la revendication 6, dans lequel l'extrémité ouverte (160) est parallèle à la direction de la longueur (L5) de la bande de nettoyage (104) ou l'extrémité ouverte (160) est perpendiculaire à la direction de la longueur (L5) de la bande de nettoyage (104).

8. Dispositif (100) selon l'une quelconque des revendications précédentes, dans lequel l'unité d'entraînement mécanique (102) comprend en outre :
un moteur électrique (126) ;
un mandrin (128), ledit mandrin (128) étant connecté de manière opérationnelle audit moteur électrique (126) ; et
un commutateur de commande (130), ledit commutateur de commande (130) étant couplé de manière opérationnelle au moteur électrique (126) pour mettre en marche ou arrêter le moteur électrique (126),
dans lequel ladite partie d'extrémité proximale (112) dudit membre rigide (108) est fixée audit mandrin (128).

9. Dispositif (100) selon la revendication 8, dans lequel ladite partie d'extrémité proximale (112) dudit membre rigide (108) est fixée de manière détachable audit mandrin (128).

10. Dispositif (100) selon l'une quelconque des revendications précédentes, comprenant en outre une source de puissance électrique (146), ladite source de puissance électrique (146) étant couplée de manière opérationnelle à ladite unité d'entraînement mécanique (102) pour entraîner le mouvement de ladite partie d'extrémité distale (110).

11. Dispositif (100) selon la revendication 10, dans lequel ladite source de puissance électrique (146) est une pile.

12. Dispositif (100) selon l'une quelconque des revendications précédentes, dans lequel ladite unité d'entraînement mécanique (102) est configurée pour être tenue à la main.

13. Dispositif (100) selon l'une quelconque des revendications précédentes, dans lequel la surface (122) de la partie d'extrémité distale (110) du membre rigide (108) est plane ou est une surface courbée.
